# EUROPEAN PATENT SPECIFICATION

(11) **EP 3 643 304 B1**
(45) Date of publication and mention of the grant of the patent: **26.03.2025**
(21) Application number: 18820046.3
(22) Date of filing: 14.02.2018
(51) Int. Cl.: A61K 9/00, A61K 9/10, A61K 9/19, A61K 47/10, A61K 47/26, A61K 31/277, A61K 31/18, A61P 31/18, B82Y 40/00, B82Y 5/00, B82B 1/00

(54) **PHARMACEUTICAL NANOSUSPENSION FOR THE THERAPY OF HIV INFECTION**
PHARMAZEUTISCHE NANOSUSPENSION ZUR THERAPIE EINER HIV-INFEKTION
NANO-SUSPENSION PHARMACEUTIQUE POUR LE TRAITEMENT DE L'INFECTION PAR LE VIH

(30) Priority: 22.06.2017 RU 2017122003
(43) Date of publication of application: 29.04.2020
(73) Proprietor: Ivachtchenko, Alexandre Vasilievich, Hallandale Beach, FL 33009 (US); Ivashchenko, Andrey Alexandrovich, Moscow 127576 (RU); Savchuk, Nikolay Filippovich, Rancho Santa Fe, CA 92067 (US); Ivachtchenko, Alena Alexandrovna, Hallandale, Florida 33009 (US); Alla Chem, LLC, Hallandale Beach, FL 33009 (US)
(72) Inventor: IVASHCHENKO, Andrey Alexandrovich, Moscow 127576 (RU); SAVCHUK, Nikolay Filippovich, Rancho Santa Fe California 92067 (US); KHVAT, Alexander Viktorovich, San Diego CA 92131 (US)
(74) Representative: Zellentin & Partner mbB Patentanwälte
(86) International application number: PCT/RU2018/000086
(87) International publication number: WO 2018/236249

(56) References cited:
- UA-C1- 26 356
- US-A1- 2005 239 881
- US-A1- 2010 063 154
- US-A1- 2010 063 154
- DEEPAK BHAMBERE ET AL: "LYOPHILIZATION / FREEZE DRYING A REVIEW", WORLD JOURNAL OF PHARMACEUTICAL RESEARCH,, vol. 4, no. 8, 1 July 2015 (2015-07-01), pages 516 - 543, XP002782505
- KUMARI KAMALA ET AL: "Nanosuspensions: A Review", INTERNATIONAL JOURNAL OF PHARMACY, vol. 7, no. 2, 1 January 2017 (2017-01-01), pages 77 - 89, XP055643073
- IZELDATELSTVO: "Passage; 37. BUFERNYYe RASTVORY (OFS 42-0072-07) [37. BUFFER SOLUTIONS (OFS 42-0072-07)]", GOSUDARSTVENNAYA FARMAKOPEYA ROSSYSKOI FEDERATSII, vol. 12, no. 1, 2008, pages 443 - 444,449, XP009518395
- ANONYMOUS: "Virom reports positive findings in phase IIB study of Elpida(r) as compared to efavirenz in combination with TDF/FTC at CROI", 17 February 2017 (2017-02-17), pages 1 - 2, XP055565841, Retrieved from the Internet <URL:https://www.prnewswire.com/news-releases/viriom-reports-positive-findings-in-phase-iib-study-of-elpida-as-compared-to-efavirenz-in-combination-with-tdfftc-at-croi-2017-300409372.html>
- ANONYMOUS: "Nadezhda dlya VICH-patsientov TASS informatsionnoe Agentstvo Rossy, Skolkovo ", 18 May 2016 (2016-05-18), pages 1 - 4, XP055660398, Retrieved from the Internet <URL:http://tass.ru/skolkovo/3291012>

## Description

### FIELD OF INVENTION

The present invention relates to a pharmaceutical composition (nanosuspension) for a long-acting injectable (LAI) drug intended for long-term supportive therapy of HIV/AIDS.

### BACKGROUND OF INVENTION

The human immunodeficiency virus (HIV) is a lentivirus (a subgroup of retroviruses) that causes an indolent disease-HIV-infection *[*Weiss R.A. How does HIV cause AIDS. Science 1993, 260 (5112), 1273-1279*.* Douek D.C., Roederer M., Koup R.A. Emerging Concepts in the Immunopathogenesis of AIDS». Annu. Rev. Med. 2009, 60, 471-84]. The human immunodeficiency virus was independently discovered in 1983 at two laboratories: one by a research team led by Luc Montagnier at the Pasteur Institute in France and the other, led by Robert Gallo at the National Cancer Institute in the United States. The findings discussing the first isolation of a new retrovirus from tissues of patients with symptoms of AIDS were published on May 20, 1983 in the journal *Science* [Barré-Sinoussi F. et al. Isolation of a T-lymphotropic retrovirus from a patient at risk for acquired immune deficiency syndrome (AIDS). Science 1983, 220 (4599), 868-871. Gallo R. C. at al. Isolation of human T-cell leukemia virus in acquired immune deficiency syndrome (AIDS). Science 1983, 220 (4599), 865-867.]. In 2008, Luc Montagnier and Françoise Barré-Sinoussi shared the Nobel Prize for Physiology and Medicine for their "discovery of the human immunodeficiency virus."

The HIV virus infects the cells of the immune system that have CD4 receptors on their surface: T helpers, monocytes, macrophages, Langerhans cells, dendritic cells, microglial cells. This leads to immunodepression and development of Acquired Immune Deficiency Syndrome (AIDS); loss of patients' bodies ability to protect themselves against infections and tumors; emergence of secondary opportunistic diseases that are not typical for people with normal immune status. Without treatment, average survival time after infection with HIV is estimated to be 9 to 11 years, depending on the HIV subtype [https://en.wikipedia.org/wiki/HIV].

According to the worldwide global statistics, in 2015, 36.7 million people globally were living with HIV, 2.1 million people were newly infected with HIV, and 1.1 million people died of AIDS-related illnesses. Since the start of the epidemic, 78 million people have become infected with HIV, of which 35 million people have died of AIDS-related illnesses.

As of December 2015, 17 million people living with HIV were receiving antiretroviral therapy, while in June 2015 this number was 15.8 million people and in 2010, 7.5 million people.

In 2015, 46% of all adults living with HIV had access to treatment, whereas in 2010, only 23%.

Since 2010, new HIV infections fell by 6%. In 2015, the global number of HIV-infected was 2.1 million people, while in 2010, 2.2 million people.

As compared to the highest figure in 2005, the rate of AIDS-associated mortality has declined by 45%, and in 2015, the number of individuals died due to AIDS worldwide was 1.1 million people against 2 million people in 2005.

HIV can be suppressed by combination antiretroviral therapy (ART) involving three or more antiretroviral drugs. ART does not cure HIV infection but suppresses viral replication within a person's body and allows an individual's immune system to strengthen and regain the capacity to fight off infections. Life expectancy for patients receiving ART may be extended to 70-80 years.

In 2016, WHO released the second edition of the "Consolidated Guidelines on the Use of Antiretroviral Drugs for Treating and Preventing HIV Infection."

In the middle of 2016, 18.2 million HIV-positive people, i.e. 46% [43-50%], worldwide were receiving ART.

Based on WHO's new recommendations to treat all people living with HIV and the offer of antiretrovirals as additional prevention choice for people at "substantial" risk, the number of people eligible for antiretroviral treatment increases from 28 million to 36.7 million people. Expanding access to treatment is the heart of a new set of targets for 2020 with the aim to end the AIDS epidemic by 2030. [http://www.who.intlmediacentre/factsheets/fs360/ru/].

To date, considerable success has been achieved in HIV/AIDS therapy using ART effective and safe regimens for the prevention and treatment of HIV. The most advanced drug now undergoing the registration procedure at the Russian Ministry of Health is Elsulfavirine (Elpida^{®}, VM1500) for HIV/AIDS ART [(a) A. Kravchenko et al. Safety and antiviral effect of Elpida (VM-1500), a novel NNRTI (+Truvada) in treatment-naïve HIV1 infected patients at 24-48 weeks therapy. HIV Drug Therapy 2016, 23-26 October 2016, P024. Glasgo, UK. (b) R.L. Murphy et al. Elsulfavirine as Compared to Efavirenz in Combination with TDF/FTC: 48-week Study. CDRI 2017, Seattle, WA http://www.prnewswire.com/news-releases/viriom-reports-positive-findings-in-phase-iib-study-of-elpida-as-compared-to-efavirenz-in-combination-with-tdfftc-at-croi-2017-300409372.html], (c) Viriom, Inc. announced interim results from Phase 3 clinical trials with Elsulfavirine, which were presented at a large conference on HIV/AIDS in Moscow [https//www.viriom.com/press-releases/2016/12/13/viriom-announces-interim-results-from-phase-3-clinical-trials-with-elpida-were-presented-at-largest-events-on-hivaids-in-moscow.].

Elsulfavirine/Elpida/VM-1500 (1a) is the prodrug of the active compound VM-1500A (1b), a Non-Nucleoside Reverse Transcriptase Inhibitor (NNRTI), which efficiently prevents HIV-1 HXB2 strain replication in the MT-4 cell line. Average IC50 values obtained for VM-1500A (1b) on a wild-type HXB2 strain and for inhibiting the replication of HIV-1 mutant viruses containing V106A, G190A, L100I/K103N, and K103N/Y181C mutations are as follows: 1.3 ± 0.4 nM (HXB2), 1.2 ± 0.2 nM (V106A). 0.6 ± 0.6 nM (G190A), 1.3 ± 0.3 nM (L100I/K103N), and 1.3 ± 0.4 nM (K103N/Y181C). 1a and 1b, both, are disclosed in US 2005/239881 A1. 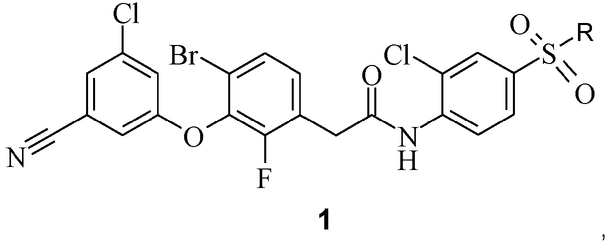 wherein R is C₂H₅CON⁻Na⁺, NH₂;

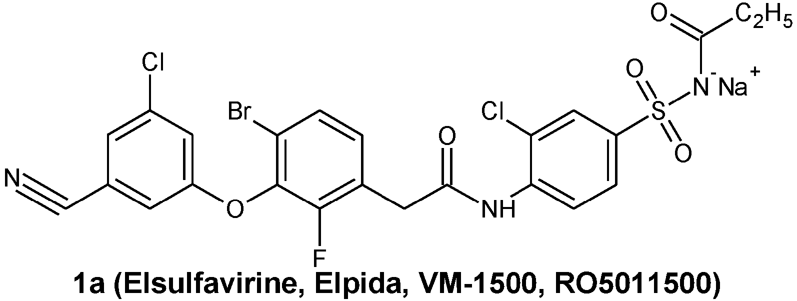

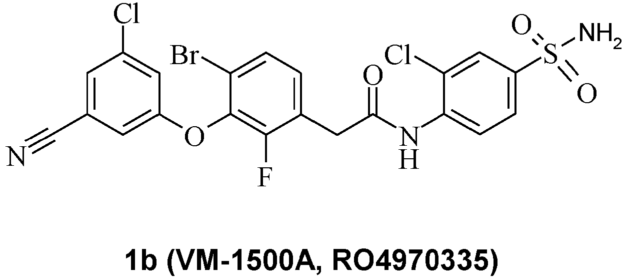

Despite the progress in the field of HIV/AIDS antiretroviral therapy, development of more effective and safe regimens for HIV prevention and treatment remains a major challenge.

In fact, this far no Long-Acting (LA) drugs for long-term supportive HIV/AIDS therapy have been registered.

The present invention relates to a pharmaceutical composition (nanosuspension) for a long-acting injectable (LAI) drug for long-term supportive therapy of HIV/AIDS.

Listed below are definitions of various terms used to describe this invention. These definitions apply to the terms as they are used throughout this specification and claims, unless otherwise limited in specific instances, either individually or as part of a larger group.

The term "active ingredient" (drug substance) refers to a physiologically active compound of synthetic or other (biotechnological, plant, animal, bacterial,and so on) origins, which exhibits pharmacological activity and is an active ingredient of a pharmaceutical composition.

The term "crystalline form" refers to a substance structure wherein the molecules are arranged to form a crystal lattice.

The term "polycrystalline form" refers to a polycrystalline substance structure consisting of a plurality of monocrystals, or crystallites of certain crystalline form.

The term "medicinal drug" refers to a compound (or a mixture of compounds forming a pharmaceutical composition) in the form of tablets, capsules, injections, ointments, or other finished dosage forms intended for the restoration, improvement, or modification of physiological functions in humans and animals, and for the treatment and prophylaxis of diseases, for diagnostics, anesthesia, contraception, cosmetology, etc.

The term "pharmaceutical composition" refers to a composition comprising the compound of general formula 1 and at least one of the components selected from the group consisting of pharmaceutically acceptable and pharmacologically compatible fillers, solvents, diluents, carriers, excipients, distributing, and receptive agents, delivery agents such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. A pharmaceutical composition for peroral, sublingual, transdermal, intramuscular, intravenous, subcutaneous, and local or rectal administration of the active ingredient, alone or in combination with another active compound, may be administered to animals and people in a standard administration form as a mixture with traditional pharmaceutical carriers. Suitable standard administration forms include peroral forms, such as tablets, gelatin capsules, pills, powders, granules, chewing gums, and peroral solutions or suspensions; sublingual and transbuccal administration forms; aerosols; implants; local, transdermal, subcutaneous, intramuscular, intravenous, intranasal, or intraocular forms; and rectal administration forms.

The term "inert filler" as used herein refers to a compound that is used for forming a pharmaceutical composition and is, as a rule, safe, nontoxic, and neither biologically nor otherwise undesirable, and comprises excipients acceptable for veterinary and human pharmaceutical use. Compounds of this invention may be administered individually but are generally administered in a mixture with one or more pharmaceutically acceptable excipients, diluents, or carriers chosen depending on the contemplated route of drug administration and standard pharmaceutical practice.

The term "therapeutically effective amount," as used herein, refers to an amount of a substance, prodrug, or drug needed for alleviating the symptoms of the disease in the subject. The dose of a substance, prodrug, or drug will meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all values therebetween, both in monotherapy and/or combination therapy. The preferred daily dose is around 0.1-7 g. As a rule, in order to quickly alleviate or eliminate the virus, a higher loading dose is given at the beginning of treatment with a subsequent reduction of the dose to a level sufficient to prevent an infection burst.

The term "subject" refers to a mammal, preferably a human being. It is assumed that a subject's treatment method may involve any of the prodrugs of general formula 1, its isotopically enriched analogs, pharmaceutically acceptable salts, hydrates, solvates, and crystalline or polymorphic forms or their combinations with other compounds and drugs for combination retroviral therapy of HIV/AIDS.

### SUMMARY OF THE INVENTION

The inventors have surprisingly found that a pharmaceutical nanosuspension comprising, as an active ingredient, a compound of general formula 1 in a crystalline or polycrystalline form is effective as an injective drug for long-term supportive therapy of HIV infection.

The subject matter of the present invention is a lyophilized nanocomposition comprising, as an active ingredient, a compound of formula 1b in a crystalline or polycrystalline form in a therapeutically effective amount, in combination with a solubilizer being poloxamer P338 and a detergent being mannitol or saccharose.

More preferable is a lyophilized nanocomposition with particle size varying from 200 nm to 900 nm.

A further subject matter of the present invention is a pharmaceutical nanosuspension comprising the nanocomposition described above, a Phosphate Buffered Saline (PBS), and water for injection for long-term supportive therapy of HIV/AIDS-infected individuals.

A still further subject matter of the present invention is a method for producing a lyophilized nanocomposition, said method consisting in the rotary grinding by means of zircon sand in an aqueous solution of the poloxamer of the compound of general formula 1 in a crystalline or polycrystalline form, a solubilizer and detergent with subsequent separation of the zircon sand and lyophilization of the resulting suspension.

Yet another subject matter of this invention is a method for producing a pharmaceutical nanosuspension by means of mixing the lyophilized nanocomposition described above and water for injection.

This invention is illustrated with drawings.
Fig. 1. Particle size measurement curves for three samples following 24-hour grinding with saccharose.
Fig. 2. Particle size measurement curves for three samples following 24-hour grinding with mannitol.
Fig. 3. Particle size measurement curves for three samples following 24-hour grinding and 20-hour settling (saccharose).
Fig. 4. Particle size measurement curves for three samples following 24-hour grinding and 20-hour settling (mannitol).
Fig. 5. Particle distribution measurement curves for three samples from Lot 740-1-101.1 (saccharose) following lyophilization and resuspending.
Fig. 6. Particle distribution measurement curves for three samples from Lot 740-1-101.2 (mannitol) following lyophilization and resuspending.
Fig. 7. Concentration-time curves VM-1500A in canine plasma for single-dose SC and IM administration of pharmaceutical nanosuspensions VM-1500-LAI and VM-1500A-LAI (10 mg/kg).

### PREFERRED EMBODIMENT

The present invention will now be described in terms of certain embodiments which are not intended to limit its scope. On the contrary, the present invention covers all alternatives, modifications, and equivalents that can be included within the scope of the claims. Thus, the following examples, which include specific embodiments, will illustrate this invention without limiting it.

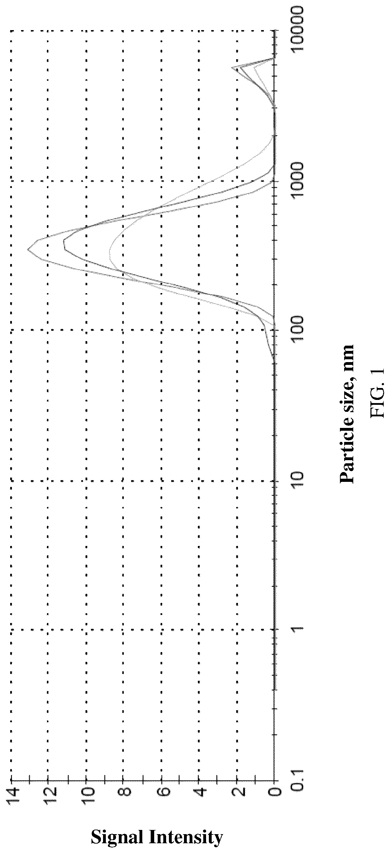

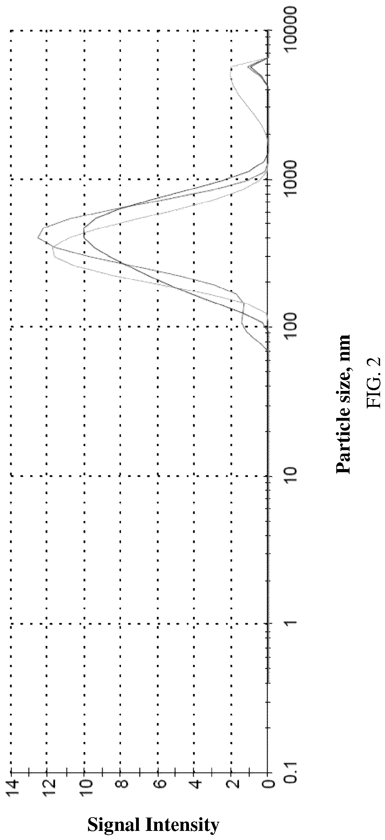

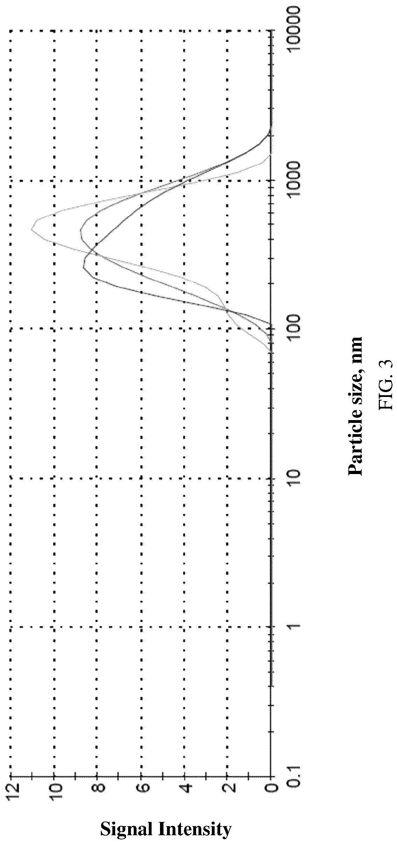

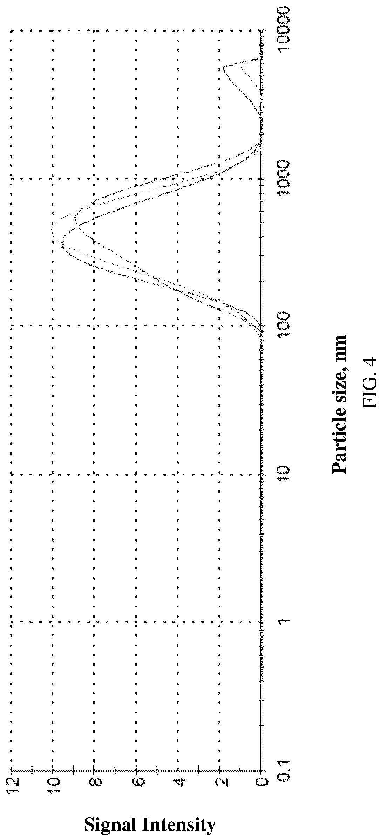

Example 1. Method for producing a lyophilized nanocomposition. Zircon sand (150 ml) (grinding media: 0.5 mm YTZ^{®} Zirconia Grinding and Dispersion Media; Tosoh USA, Inc.) and a solution of poloxamer P338 (10.0 g) and saccharose or mannitol (11.6 g) in 400 ml of PBS (pH = 7.4) were placed into a jar. The resulting solution (150 ml) was placed into a jar, and zircon sand (150 ml) (grinding media: 0.5 mm YTZ^{®} Zirconia Grinding and Dispersion Media; Tosoh USA, Inc.) and compound VM1500A (15.07 g) were added. The jar was placed on a mill (U.S. Stoneware 700 Series jar mill), where the mixture was ground for 24 hours at 104 rev/min. The end of the process was checked by particle distribution analyses on the Malvern Zetasizer Nano ZS instrument. Upon completion of the process, the rotation was stopped and the mixture was allowed to stand for 16-20 hours at 2-8°C to let the zircon sand settle. The suspension was filtered through a glass filter (filtration pore diameter: 5-15 µm) and analyzed for concentration of the compound VM1500A (92-97% from nominal) to afford a 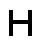 ananosuspension, which was aseptically poured into 5-ml sterile glass vials (2 ml each), frozen, and lyophilized to afford a lyophilized nanocomposition with characteristics given in Tables 1 and 2 and Figs. 1, 2, 3, and 4.

**Table 1. Particle size in the nanosuspension after grinding for 24 h (the mean of three measurements)**

| Sample | Sample volume, µl | Particle size, nm | PDI* | Quality |
|---|---|---|---|---|
| Lot No. 740-1-101.1 (saccharose) | 30 | 442.3 | 0.428 | good |
| Lot No. 740-1-101.1 (saccharose) | 10 | 357.7 | 0.283 | good |
| Lot No. 740-1-101.2 (mannitol) | 30 | 395.6 | 0.31 | good |
| Lot No. 740-1-101.2 (mannitol) | 10 | 552.4 | 0.487 | good |

| | | | | |
|---|---|---|---|---|
| *PDI is here and elsewhere a dispersion index, which characterizes the uniformity of particles in the mixture. | | | | |

**Table 2. Particle size in the nanosuspension after grinding for 24 h and settling for 20 h (the mean of three measurements)**

| Sample | Sample volume, µl | Particle size, nm | PDI | Quality |
|---|---|---|---|---|
| Lot No. 740-1-101.1 (saccharose) | 30 | 402.9 | 0.367 | good |
| Lot No. 740-1-101.1 (saccharose) | 10 | 362.1 | 0.268 | good |
| Lot No. 740-1-101.2 (mannitol) | 30 | 403.5 | 0.28 | good |
| Lot No. 740-1-101.2 (mannitol) | 10 | 381.3 | 0.286 | good |

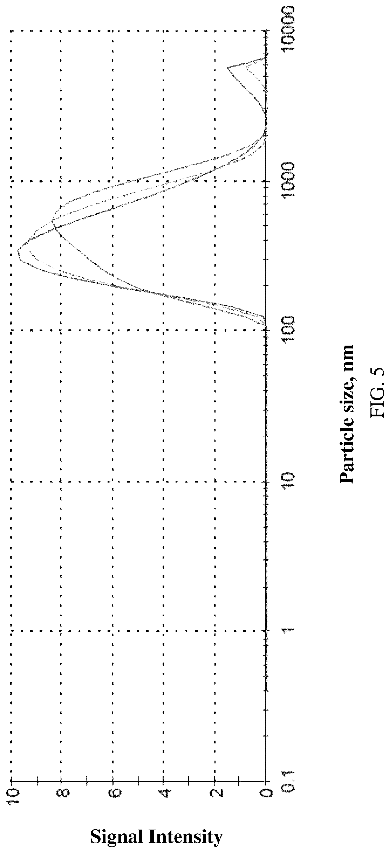

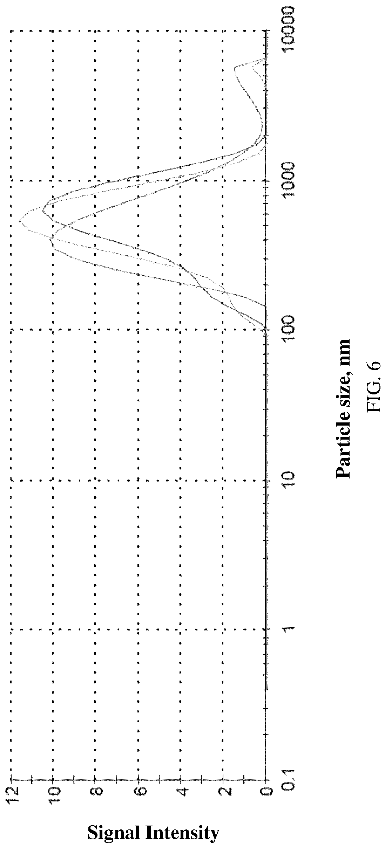

Example 2. Method for producing a pharmaceutical nanosuspension. To the nanosuspension obtained in Example 1 was added a pre-prepared sterile PBS solution (pH = 6.8) based on 2.2 ml per 5-ml vial to afford a ready-to-use pharmaceutical nanosuspension with characteristics given in Table 3 and Figs. 5 and 6.

**Table 3. Particle distribution in the pharmaceutical nanosuspension**

| Pharmaceutical nanosuspension | | Lot No. 740-1-101.1 (saccharose) | Lot No. 740-1-101.2 (mannitol) |
|---|---|---|---|
| pH | | 7.44 | 7.29 |
| Particle distribution (10-µl sample) | Particle size, nm | 381.7 | 447.6 |
| | PDI | 0.255 | 0.248 |
| Particle distribution (30-µl sample) | Particle size, nm | 386.1 | 442.6 |
| | PDI | 0.252 | 0.252 |
| HPLC analysis (percent of the nominal value) | | 93.5% | 95.0% |

Example 3. Pharmacokinetics of pharmaceutical nanosuspensions VM1500LAI and VM1500A-LAI in dogs.

The nanosuspensions VM1500LAI and VM1500A-LAI obtained in Example 2 were injected to Beagle dogs subcutaneously and intramuscularly as a single dose of 10 mg/kg, and the content of VM1500 in canine plasma was measured for 120 days after the injections. The concentration of tested compounds was measured using the HPLC-MS/MS method.

To administer the dose, pre-prepared nanosuspensions were injected to the dogs subcutaneously or intramuscularly at 0.1 ml/kg in the same spot. The blood was collected 0, 0.5, 1, 2, 4, 8, 24 h and 2, 3, 6, 8, 12, 15, 18, 22, 29, 36, 43, 50, 57, 64, 71, 78, 85, 92, 107, 114 and 121 days after the injection. The whole blood for plasma production was collected in test tubes containing sodium heparin. Immediately after collecting blood the test tube was cautiously turned over 8 times for better mixing of blood and heparin sodium and then incubated for 15 minutes at room temperature. The plasma was then separated by centrifugation at 3000 rev/min for 10 min. A plasma aliquot of 1 ml was carefully transferred into a cryovial using a self-filling pipette while avoiding contact between the tip and the cell mass / barrier gel. The plasma samples from one Vacuette tube were transferred into two cryovials. The resulting samples were frozen. Prior to analysis, plasma samples and blood cells should be kept at -80°C.

The concentration of VM-1500 and VM-1500A was measured by means of HPLC-MS/MS. The compound VM-6819 having a similar structure was used as the internal standard (IS). Scanning in a total ion current mode (MS1) allowed us to identify a molecular ion for each tested compound and IS, while basic product ions were recorded in the MS2 mode. To attain maximum sensitivity in the quantitative analysis, the MS/MS procedure was optimized in the MRM mode. For each analyte, at least two MRM transitions were recorded, and one of them was used in quantitative chromatogram processing.

To achieve the best chromatographic parameters, suitable conditions were chosen for the injection of the test compound and IS in a mixture of MeCN:H2O (1:1) into the MS/MS detector via an HPLC system. Calibration standards for VM1500 and VM1500A in plasma were prepared in a concentration range of 1.0-2000.0 ng/ml. Along with calibration standards, a zero sample (k0) and k0 with IS were prepared as well as two series of control samples (QC) for low, medium, and high concentrations to serve as the indication of the quality of quantitative calculations. From the stock solutions of VM1500 (2.0 mg/ml) and VM1500A (2.0 mg/ml) in DMSO, 10-fold work dilutions in MeCN:H2O (1:1) were prepared. Solutions for the calibration standards of (k) and QC samples were prepared from single aliquots of the VM1500 and VM1500A stock solutions.

Plasma experimental samples used for the preparation of standard samples were thawed at room temperature, stirred and centrifuged for 5 min at 14000 rev/min. Intact plasma (90 µl) was transferred into a 1.5-ml test tube, and 10 µl of a 10-fold standard dilution of the mixture of test compounds was added to the acetonitrile : water (1:1) mixture. To the experimental sample (90 µl), a pure acetonitrile : water (1:1) mixture (10 µl) was added. The samples were thoroughly mixed for 10 seconds using a vortex mixer. Then, 10 µl of IS (VM-6819 5 mg/ml in acetonitrile : water 1:1) was added, and the mixture was vortexed for another 10 seconds. To the samples, 1 ml of the ethyl acetate : hexane (60:40) mixture was added and mixed in a vortex mixer for 20 sec and then on a shaker at 1000 rev/min for 5 min. The samples were centrifuged at 14 000 rev/min for 10 min.

The organic phase (0.8 ml) was taken to 1.5-ml test tubes and dried under nitrogen flow in an evaporator at 40°C. Dry extracts were diluted in 150 µl of the acetonitrile : water (1:1) mixture, were vortexed for 10 sec and transferred into a clean plate for an HPLC/MS/MS analysis.

The concentrations of VM1500 and VM1500A in the samples were estimated from the calibration curve plotted from chromatographic peak areas of the standard samples VM1500 and VM1500A in a matrix (plasma or blood cells) normalized to the IS area. The calibration curve was plotted by fitting a simplest model adequately describing analytical signal versus concentration relationship and using a suitable normalization. Basic pharmacokinetic parameters were found by a model-independent method using the WinNonlin Professional 6.3 software (Pharsight Corporation) based on experimentally derived concentration-time data for each animal:
- Cₘₐₓ - maximum concentration in plasma;
- Tₘₐₓ - time to reach maximum concentration in plasma;
- AUC₀₋ₜ - area under the PK curve from the moment of drug administration to the last point of concentration measurement;
- AUC_{0-inf}- area under the PK curve from the moment of drug administration to infinity;
- T_{1/2} - plasma elimination half-life;
- kₑₗ - elimination rate constant (parameter characterizing the rate of drug elimination from the plasma);
- MRT - middle residence time from the moment of drug administration.

Statistical processing of the results was performed by means of descriptive statistics. The following parameters were calculated: the arithmetic mean value (M), the standard deviation (SD), the standard error of the mean (SEM), the coefficient of variation (CV), the median. Statistical analysis was carried out using the WinNonlin Professional 6.3 software (Pharsight Corporation).

Table 4 summarizes pharmacokinetic parameters characterizing the behavior of VM-1500 and VM-1500A after subcutaneous (SC) and intramuscular (IM) injections of the nanosuspensions VM-1500-LAI and VM-1500A-LAI to dogs.

**Table 4. Pharmacokinetic parameters VM-1500 and VM-1500A in canine plasma after SC and IM injections of the nanosuspensions VM-1500-LAI and VM-1500A-LAI in a dose of 10 mg/kg**

| Paramet er | Unit | SC | IM | SC | | IM | |
|---|---|---|---|---|---|---|---|
| | | VM1500-LAI | | VM 1500-LAI | VM 1500A-LAI | VM 1500-LAI | VM1500A -LAI |
| | | VM-1500 | | VM-1500A | | | |
| **AUC_{inf}** | h*ng/ml | 4435.7 | 5 743.6 | 89210.0 | 89 409 | 121 175.1 | 83916 |
| **AUCₗₐₛₜ** | h*ng/ml | 3 989.9 | 5596.7 | 88 830.4 | 78 492 | 120 975.5 | 79 518 |
| **Cmax** | ng/ml | 111.4 | 1 158.7 | 558.7 | 104 | 1 225.0 | 153 |
| **T_{1/2}** | h | 229 | 74.9 | 170 | 581 | 153 | 446 |
| **Kel** | 1/h | 0.003 | 0.009 | 0.004 | 0.001 | 0.005 | 0.002 |
| **MRT_{inf}** | h | 180 | 38.6 | 205 | 957 | 90 | 657 |
| **MRTₗₐₛₜ** | h | 95 | 29.1 | 200 | 671 | 88 | 537 |
| **Tmax** | h | 1.7 | 1.0 | 64 | 216 | 32 | 64.0 |

With intramuscular injection, higher Cmax values were observed both for VM-1500 and its metabolite VM-1500A than with subcutaneous injection. For the VM-1500A-LAI formulation, however, the difference was not so appreciable. Furthermore, the total exposure of VM-1500 and VM-1500A in the case of intramuscular injection of VM-1500-LAI was higher than in the case of subcutaneous one, whereas the exposure of VM-1500A was independent of the way of administration of the VM-1500A-LAI formulation. It is to be noted that with intramuscular injection, VM-1500A achieves maximum concentration earlier than with subcutaneous one. Half-life does not substantially depend on the way of administration.

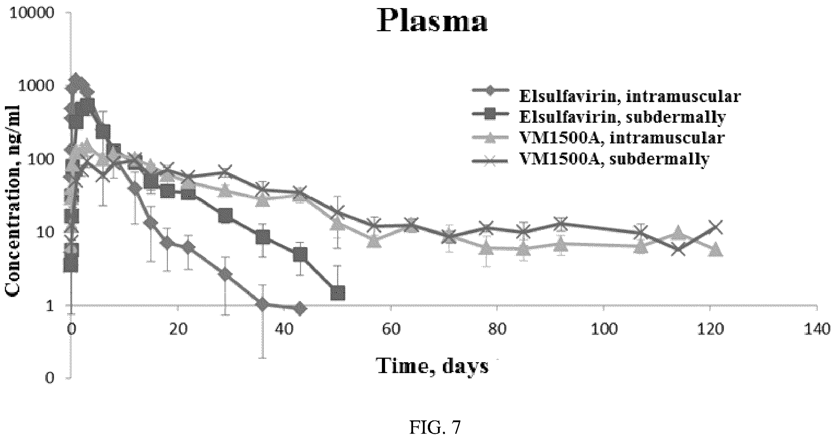

Average VM-1500A concentrations (C) in canine plasma after single SC and IM injections of the nanosuspensions VM1500-LAI and VM1500A-LAI are given in Table 5 and in Fig. 7.

**Table 5. Concentration of VM-1500A in canine plasma after single SC and IM injections of the depot formulations VM-1500-LAI and VM-1500A-LAI at 10 mg/kg**

| Time | | VM1500-LAI IM | | VM1500-LAI SC | | VM1500A-LAI IM | | VM1500A-LAI SC | |
|---|---|---|---|---|---|---|---|---|---|
| hours | day s | Mean, ng/ml | SD, ng/ml | Mean, ng/ml | SD, ng/ml | Mean, ng/ml | SD, ng/ml | Mean, ng/ml | SD, ng/ml |
| 0 | 0.0 | 0.00 | 0.00 | 0.00 | 0.00 | 0.00 | 0.00 | | |
| 1 | 0.0 | 55.67 | 7.76 | 3.54 | 0.58 | 6.35 | 1.59 | 3.40 | 3.48 |
| 1 | 0.0 | 129.67 | 12.74 | 5.62 | 1.56 | 11.96 | 6.64 | 4.24 | 2.56 |
| 2 | 0.1 | 350.67 | 74.54 | 16.57 | 2.75 | 28.80 | 9.30 | 7.12 | 4.59 |
| 4 | 0.2 | 482.33 | 87.93 | 31.43 | 6.11 | 40.23 | 3.82 | 12.25 | 5.03 |
| 8 | 0.3 | 892.67 | 108.49 | 77.93 | 8.47 | 84.43 | 25.20 | 16.43 | 3.98 |
| 24 | 1.0 | 1166.67 | 151.85 | 314.67 | 35.64 | 124.50 | 11.46 | 49.80 | 13.17 |
| 48 | 2.0 | 1023.00 | 187.20 | 480.00 | 155.79 | 131.67 | 4.25 | 70.23 | 16.42 |
| 72 | 3.0 | 812.67 | 28.36 | 535.33 | 210.24 | 151.33 | 18.06 | 91.73 | 5.61 |
| 144 | 6.0 | 245.33 | 51.60 | 232.67 | 8.14 | 99.37 | 22.09 | 59.96 | 4.82 |
| 192 | 8.0 | 105.70 | 26.49 | 129.00 | 16.70 | 115.50 | 12.26 | 86.97 | 3.57 |
| 288 | 12.0 | 39.43 | 9.03 | 90.27 | 10.83 | 101.22 | 8.75 | 96.22 | 24.58 |
| 360 | 15.0 | 12.97 | 4.16 | 48.40 | 7.41 | 79.38 | 22.07 | 57.62 | 4.52 |
| 432 | 18.0 | 7.06 | 2.98 | 36.10 | 5.20 | 61.52 | 7.52 | 71.20 | 4.98 |
| 528 | 22.0 | 6.04 | 1.89 | 34.30 | 3.70 | 48.85 | 7.19 | 56.42 | 9.05 |
| 696 | 29.0 | 2.61 | 0.84 | 16.80 | 4.11 | 37.07 | 3.26 | 65.5 | 10.8 |
| 864 | 36.0 | 1.03 | 0.10 | 8.57 | 2.31 | 27.80 | 7.89 | 37.8 | 3.1 |
| 1032 | 43.0 | 0.89 | 0.18 | 4.83 | 1.98 | 32.37 | 5.09 | 34.5 | 12.5 |
| 1200 | 50.0 | BLQ | | 1.47 | 0.70 | 13.30 | 1.31 | 18.4 | 3.9 |
| 1368 | 57.0 | BLQ | | BLQ | | 7.6 | 2.55 | 12.02 | 2.48 |
| 1536 | 64.0 | BLQ | | BLQ | | 12.27 | 3.52 | 12.40 | 1.60 |
| 1704 | 71.0 | BLQ | | BLQ | | 8.88 | 2.74 | 8.53 | 1.12 |
| 1872 | 78.0 | BLQ | | BLQ | | 6.03 | 1.84 | 11.24 | 3.41 |
| 2040 | 85.0 | BLQ | | BLQ | | 5.87 | 2.07 | 9.92 | 2.52 |
| 2208 | 92.0 | BLQ | | BLQ | | 6.81 | 1.46 | 12.79 | 3.01 |
| 2568 | 107 | BLQ | | BLQ | | 6.43 | 1.38 | 9.59 | 0.39 |
| 2736 | 114 | BLQ | | BLQ | | 9.68 | 1.33 | 5.72 | 1.12 |
| 2904 | 121 | BLQ | | BLQ | | 5.70 | 1.81 | 11.47 | 1.07 |

| | | | | | | | | | |
|---|---|---|---|---|---|---|---|---|---|
| BLQ - Below Limit of Quantification | | | | | | | | | |

As can be seen from Table 5 and Fig. 7, after IM injection of the pharmaceutical nanosuspension VM-1500-LAI in a dose of 10 mg/kg to dogs, the effective concentration of the drug VM1500A (IC50 = 1.3 nM or 0.74 ng/ml) in plasma is maintained for about a month (after 29 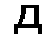 days, CVM-1500A = 2.61 ng/ml), whereas after SC injection, for about a month and a half (after 43 days, CVM-1500A = 4.83 ng/ml).

An even more convincing result was observed after the IM and SC injections of the nanosuspension VM-1500-LAI in a dose of 10 mg/kg to dogs. In this case, the effective concentration of VM1500A maintained for four months (after 121 days, CVM-1500A = 5.70 ng/ml after IM injection and CVM-1500A =11.47 ng/ml after SC injection).

### INDUSTRIAL APPLICABILITY

The invention could be used in medicine and veterinary.

## Claims

1. A lyophilized nanocomposition comprising, as an active ingredient, a compound of formula 1b in a crystalline or polycrystalline form 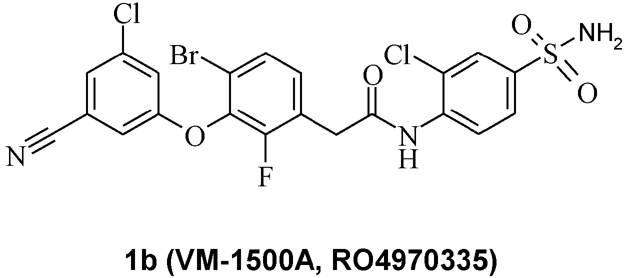
in a therapeutically effective amount,
in combination with a solubilizer being poloxamer P338 and a detergent being mannitol or saccharose.

2. The composition according to Claim 1 additionally comprising a pharmaceutically acceptable filler, additive or diluent.

3. The composition according Claim 1 or 2 for the production of a pharmaceutical nanosuspension.

4. A pharmaceutical nanosuspension as an injectable drug for long-term maintenance therapy of an HIV infection, comprising the composition according to any one of Claims 1-3, a phosphate buffered saline (PBS), and water for injection.

5. A method for producing a composition according to any one of Claims 1-3, comprising rotary grinding using zircon sand in an aqueous solution of poloxamer P338 as a solubilizer of the compound of formula 1b in crystalline or polycrystalline form, and a detergent selected from mannitol or saccharose, followed by separation of the zircon sand and lyophilization of the obtained suspension.

6. A method for producing a pharmaceutical nanosuspension by mixing a composition according to any one of Claims 1-3, a phosphate buffered saline (PBS) having pH = 6.8 and water for injection.

## Patentansprüche

1. Lyophilisierte Nanozusammensetzung, umfassend als Wirkstoff eine Verbindung der Formel 1b in kristalliner oder polykristalliner Form 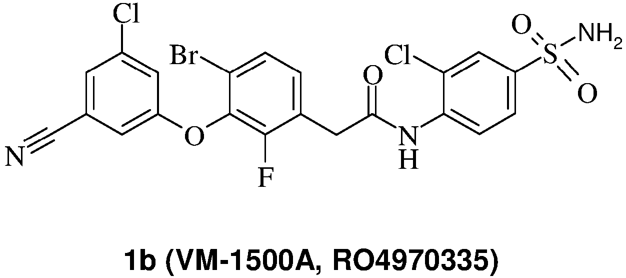
in einer therapeutisch wirksamen Menge,
in Kombination mit einem Lösungsvermittler, der Poloxamer P338 ist, und einem Detergens, das Mannitol oder Saccharose ist.

2. Zusammensetzung nach Anspruch 1, die zusätzlich einen pharmazeutisch annehmbaren Füllstoff, Zusatzstoff oder Verdünnungsmittel umfasst.

3. Zusammensetzung nach Anspruch 1 oder 2 zur Herstellung einer pharmazeutischen Nanosuspension.

4. Pharmazeutische Nanosuspension als injizierbares Arzneimittel zur langfristigen Erhaltungstherapie einer HIV-Infektion, umfassend die Zusammensetzung nach einem der Ansprüche 1 bis 3, eine phosphatgepufferte Kochsalzlösung (PBS) und Wasser für Injektionszwecke.

5. Verfahren zur Herstellung einer Zusammensetzung nach einem der Ansprüche 1 bis 3, umfassend das Rotationsmahlen der Verbindung der Formel **1b** in kristalliner oder polykristalliner Form unter Verwendung von Zirkonsand in einer wässrigen Lösung von Poloxamer P338 als Lösungsvermittler und eines Detergens, ausgewählt aus Mannit oder Saccharose, gefolgt von Abtrennen des Zirkonsands und Lyophilisieren der erhaltenen Suspension.

6. Verfahren zur Herstellung einer pharmazeutischen Nanosuspension durch Mischen einer Zusammensetzung nach einem der Ansprüche 1-3, einer phosphatgepufferten Kochsalzlösung (PBS) mit einem pH-Wert von 6,8 und Wasser für Injektionszwecke.

## Revendications

1. Nanocomposition lyophilisée comprenant, en tant que principe actif, un composé de formule **1b** sous forme cristalline ou polycristalline 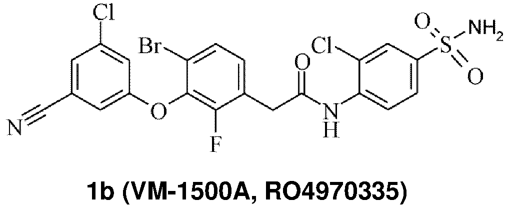
dans une quantité thérapeutiquement efficace,
en combinaison avec un solubilisant étant le poloxamère P338 et un détergent étant le mannitol ou le saccharose.

2. Composition selon la revendication 1 comprenant en outre une charge, un additif ou un diluant pharmaceutiquement acceptable.

3. Composition selon la revendication 1 ou 2 pour la production d'une nanosuspension pharmaceutique.

4. Nanosuspension pharmaceutique sous forme de médicament injectable pour le traitement d'entretien à long terme d'une infection par le VIH, comprenant la composition selon l'une quelconque des revendications 1 à 3, une solution saline tamponnée au phosphate (PBS) et de l'eau pour injection.

5. Procédé de production d'une composition selon l'une quelconque des revendications 1 à 3, comprenant le broyage rotatif à l'aide de sable de zircon dans une solution aqueuse de poloxamère P338 en tant que solubilisant du composé de formule **1b** sous forme cristalline ou polycristalline, et d'un détergent choisi parmi le mannitol ou le saccharose, suivi de la séparation du sable de zircon et de la lyophilisation de la suspension obtenue.

6. Procédé de production d'une nanosuspension pharmaceutique par mélange d'une composition selon l'une quelconque des revendications 1 à 3, d'une solution saline tamponnée au phosphate (PBS) ayant un pH = 6,8 et d'eau pour injection.
